(19) Europäisches Patentamt
European Patent Office
Office européen des brevets

(11) Publication number: 0 230 702 A1

(12) # EUROPEAN PATENT APPLICATION

(21) Application number: 86300434.7

(22) Date of filing: 22.01.86

(51) Int. Cl.[4]: C12N 15/00 , C12P 21/02 , D01D 4/00

(43) Date of publication of application: 05.08.87 Bulletin 87/32

(84) Designated Contracting States: AT BE CH DE FR GB IT LI LU NL SE

(71) Applicant: P A Consulting Services Limited
Hyde Park House 60a Knightsbridge
London SW1X 7LE(GB)

(72) Inventor: Petty-Saphon, Satham
Blythburgh House Wendens Ambo
Saffron Walden, Essex CB11 4JU(GB)
Inventor: Light, Janice Ann
12 Granby Road Stevenage
Hertfordshire, SG1 4AR(GB)

(74) Representative: Matthews, Heather Clare et al
Keith W Nash & Co Pearl Assurance House
90-92 Regent Street
Cambridge CB2 1DP(GB)

(54) **Improvements in or relating to production of silk.**

(57) Silk proteins are produced using a recombinant microorganism for producing the protein fibroin and possibly also the protein sericin, for spinning to produce silk. The relevant protein(s) may also be produced using tissue culture techniques. The invention may be used to produce material identical to natural silk proteins. Alternatively, deliberately modified proteins may be produced, for production of silks having modified properties not necessarily found in nature.

EP 0 230 702 A1

# Improvements in or relating to production of silk

## Field of the Invention

This invention relates to the production of silk protein, that is protein components of silk.

## Background to the Invention

Silk is produced naturally by the larvae of the Bombyx mori moth and many other Arthropods. The Bombyx mori moth larva synthesises the components of silk inside its body and extrudes them in the form of a fibre of silk. The silk fibre consists of twin filaments of the protein fibroin which are cemented or glued together by the protein sericin. These moth larvae are used as the basis of commercial silk production, and produce fibres with a composition and structure that result in natural silk having a unique combination of properties of strength, softness and lustre, which cannot be matched by conventional manmade fibres.

Natural production of silk from these moth larvae is, however, limited both in quality by geographical, political and other factors. As a result, the supply of natural silk is limited and prices are high.

Silks are also produced by a wide range of other Arthropods, for purposes such as:
-trapping prey (eg spider's webs and other prey-catching devices)
-use in cocoons
-use in egg cases
-use in burrow construction
-camouflaging body-armour

Spider silks are of particular interest. For example the orb-web spiders, such as Araneus diadematus, produce 5 different types of silk having different physical properties for different purposes, to act as:

1. drag-line and web framework
2. viscid catching net of the web
3. fibrous discs attaching web to support
4. a cocoon for prey, once caught
5. materials for the constructions of egg-cases.

These materials are of interest because of the wide variety of physical properties which are associated with different types of silk. In many cases silks with interesting properties cannot be harvested commercially because of the difficulties of culturing the organisms under controlled conditions and of harvesting the material.

It is an object of the invention to provide a method for the production of components of silk which does not involve the Bombyx mori moth larva, or other Arthropods.

It is also an object of the invention to produce components of novel silks having specific properties, not necessarily found in naturally produced silks, and the terms "silk" and "silk protein" as used in this specification should be construed accordingly to cover such materials.

## The Invention

According to one aspect of the invention, there is provided a method of producing components of silk, comprising producing from a recombinant microorganism the protein fibroin for spinning to produce silk.

The method may also involve producing the protein sericin from a recombinant microorganism (either the same microorganism which produces fibroin, or a different microorganism), for spinning together with fibroin. However, in many cases sericin is not required, because of the nature of the industrial spinning process used.

In Bombyx mori, and possibly also in other arthropods, the fibroin protein consists of two polypeptides, one very large and one small, joined together. References to fibroin in this specification should be construed to mean either the large polypeptide or the fibroin protein, as appropriate.

The protein or proteins produced need not be identical to fibroin or sericin, and similar proteins may also produce silk provided they are sufficiently fibroin-like or sericin-like as appropriate. References to fibroin and sericin should be construed accordingly. Further, by producing deliberately modified proteins, by suitable genetic engineering of the microorganism(s), components may be produced for silks having modified properties. In this way materials having specified properties suited to particular applications may be produced.

Microorganisms may be genetically modified to produce the required protein(s), fibroin and possibly also sericin, by conventional genetic engineering techniques. For example, genes encoding for fibroin (and possibly also sericin) could be generated or isolated, and inserted into a suitable host vector. The genes could be produced by reverse transcription of Bombyx mori messenger RNA to produce complementary DNA, or by the construction of a Bombyx mori gene bank. A construction of an artificial silk gene from oligonucleotides, according to the known amino acid sequences of the silk proteins is a further possibility also included in the scope of this present invention, as is the use of genes derived from other silk providing organisms.

In any case it may be necessary to modify the DNA sequence coding for the silk gene to make production in the host organism of choice efficient and reliable.

A recombinant mircoorganism capable of producing sericin may be produced by a similar technique.

The cloned or constructed gene is expressed in a suitable host microorganism using an appropriate vector and promoter system. Export from the microorganism of the silk peptides will be arranged, if possible. Examples of possible host microorganisms include:

Eschericia coli
Saccharomyces cerevisiae
Pseudomonas spp
Rhodopseudomonas spp
Bacillus subtilis and other Bacilli
Streptomyces coelicolor and other Streptomycetes
Methylomonas spp

Fibroin and sericin may be produced by separate microorganisms or together in the same organism.

Hence, in a further aspect, the present invention provides a recombinant microorganism which has been genetically modified to be capable of producing fibroin and/or sericin.

The relevant protein, fibroin or sericin, may be produced by such a recombinant microorganism (and extracted from the microorganism cells if produced intracellularly) by a fermentation process.

The present invention thus provides a method of producing fibroin and/or sericin, comprising utilising recombinant microorganisms which have been genetically modified to produce fibroin and/or sericin.

Silk components may also be produced using tissue culture techniques.

Hence, according to another aspect of the present invention there is provided a method of producing the components of silk such as fibroin and possibly also sericin by tissue culture techniques, for spinning to produce silk.

Any suitable animal or plant cells may be used in tissue culture techniques. For example, cells of Bombyx mori may be grown in culture. These cultures will be used to produce the silk components which can then be spun to produce the silk.

Hence, in a further, preferred, aspect the present invention provides a method of growing cells of Bombyx mori in culture capable of producing fibroin.

The invention also provides a method of growing cells of Bombyx mori in culture capable of producing sericin.

The relevant polypeptides (fibroin, and possibly also sericin or other similar materials) may be treated and spun to produce a fibre or other organised structure similar or indistinguishable in properties to natural silk, or deliberately having modified properties, as noted above, Thus, the invention can be used for the production of modified or designed silks having specified properties, not necessarily found in Nature, eg designed for particular industrial materials applications.

This invention also covers the production of silk components such as the polypeptides fibroin or sericin when produced by the relevant methods of the invention.

Examples of some of the preferred expressions of the Invention

Genetic engineering techniques, combined with synthetic oligonucleotide chemistry, can be used to produce molecules very similar to natural silk proteins in a novel biological host. This involves the following steps:

a) Identifying the silk (protein) of interest, and the naturally producing animal, eg Arthropod, of interest.

b) Cloning the silk gene of interest from the Arthropod of interest into a bacterial host such as E.coli. The gene contains the information which defines the protein product.

c) Elucidating the nucleotide sequence of the gene.

d) Adapting this gene so that it can be readily expressed (read, and translated into protein) and stably inherited in the chosen final host. This is done by changing the nucleotide sequence, substituting synthetic DNA where necessary.

e) Providing the gene with a signal (promoter and translation start signal) which tells the final host organism that this gene should be expressed (the protein product should be made) efficiently under certain conditions.

The silk gene for use in a microbial host should probably be considerably shorter than the Bombyx mori silk gene, as microbes do not express very large genes efficiently.

f) Transferring the engineered silk gene to the final host organism, and developing a process for the large-scale production of the silk protein.

g) Developing methods for the recovery of silk protein for spinning it into fibres or otherwise processing it into organised structures.

Such techniques can also be used to produce novel silk proteins with physical properties not found in nature. This involves the following:

a) Design, synthesis and cloning of a unique, novel silk gene.

b) The designed gene should have features enabling its expression in a microbial host. Its features should also be designed to give its products physical properties appropriate for a range of specific commercial application.

The sequence Gly-Ala-Gly-Ala-Gly-Ser is commonly found in natural silk fibroin protein(s), in a crystalline region of the protein. (The protein consists of up to 10 repeated crystalline (highly ordered) regions, interspersed with amorphous regions.)

This sequence may be used as the basis of a designed silk protein. For example, a designed silk protein might comprise sets of the sequence:

$(\text{Gly-Ala-Gly-Ala-Gly-Ser})_n$ interspersed with other sequences possibly forming amorphous regions.

Other repeated sequences either found in natural silks or designed de novo could also form the basis of a new silk protein.

The resulting array of repeated sequences, although possibly comprising sequences occuring in natural silks, could nevertheless constitute a distinct new entity non-identical with (and significantly different from) any protein found in Nature.

Certain naturally occuring sequences which may be detrimental to the properties sought in a particular protein may to advantage be omitted.

By varying the length of the protein product, physical properties may be varied.

Design of a gene to give a particular amino acid sequence

By way of example we will consider use of the naturally ocurring sequence Gly-Ala-Gly-Ala-Gly-Ser.

The base unit (18 nucleotides long) of a gene specifying Gly-Ala-Gly-Ala-Gly-Ser could have 6144 different nucleotide sequences. This number of variations occurs because there is more than one gene sequence (codon; set of three nucleotides) able to specify a single amino acid. In the simplest example one of these possibilities is chosen and is polymerised in vitro eg 30 to 100 fold to produce a gene of 540-1800 nucleotides in length, probably flanked by other coding sequences.

The polymerised sequence designed for use in a microbial host such as E. coli might be

GGT GCT GGT GCC GGC TCT

| | | | | |

Gly Ala Gly Ala Gly Ser

(Sequence of non-coding DNA strands)

In more sophisticated examples, two, three or four different unit sequences of eg 18 nucleotides are incorporated together in the gene, to decrease the repetitive nature of the nucleotide sequence, or to provide different properties.

In other more sophisticated examples the sequence of aminoacids in the protein is varied so that the sequence:

Ser-Gly-Ala-Ala-Gly-Tyr

might appear several times in the gene.

In even more sophisticated applications, further modifications to the silk gene sequences are made.

In one preferred example, crystalline, highly ordered (repetitive sequence) structures in a designed protein are interspersed with amorphous (unordered, non-repetitive sequence) regions.

For example, a protein:

Crystalline-Amorphous-Crystalline-Amorphous-Crystalline may be produced where the crystalline region contains $(Gly\text{-}Ala\text{-}Gly\text{-}Ala\text{-}Gly\text{-}Ser)_n$ or related sequences. The amorphous regions may contain amino acids with bulky side chains, such as proline or tryptophan which disrupt ordered structures.

The inclusion of different amorphous regions, interspersed with the crystalline regions, can confer an extra range of physical properties, such as a range of extensibilities, on a final fibrous product. Properties may also be varied by varying the length of the amorphous regions, and by varying the length of the crystalline regions.

## PROCESS FOR THE MICROBIAL PRODUCTION OF SILKS

### Control of silk production

It is important that the gene is only expressed under certain conditions, because the aim is to grow large numbers of host organisms (cells), and to extract large amounts of the silk protein. If the organisms are making silk protein while they are growing, they will grow slowly, and any organism which has lost the ability to make the silk protein (by a mutation) will grow and divide faster. Such a mutated organism type would overgrow the culture, gradually replacing the organism making the silk protein. Therefore, the organism carrying the silk gene is programmed only to make silk protein when the population of organisms is of the maximum required size. Then a signal is given such as a change in temperature, addition or exhaustion of a metabolite, or simply a slowing down of growth as the population becomes dense. This signal tells the organism to start making silk protein. Over a period of time, large amounts of silk protein are accumulated by each organism. This silk protein may be retained within the cells (intra-cellular) or secreted (extra-cellular).

It may also be important to ensure that the vector (the independently reproducing molecule) carrying the silk gene is maintained at a low concentration in each organism during the growth of the organisms, but at a signal, analogous to that described above, begins to reproduce itself, so that eventually it is present at a high concentration, enhancing the rate at which the silk product is made. This may be required to prevent slow growth of the organisms containing the silk gene, and the risk of overgrowth by mutated organisms as described above. The vector can either be inserted into the chromosome during growth of the cells or free in the cell's cytoplasm.

### Host Organism

The most common host organism is likely to be a microbe such as E.coli., a Bacillus , or Saccharomyces, and the process description is given with this in mind. However, in some cases it may be more appropriate to use other microorganisms, animal cells or plant cells.

For each host organism, a different set of criteria for design or adaptation of the silk gene must be adhered to for efficient silk protein production. This is because each type of organism has different populations of adaptors (tRNAs) for translating its genetic information into protein products, commonly described as codon usage. The gene structure should be adapted to fit as closely as possible with the tRNA population structure found in the host organism. Other requirements in terms of acceptable gene, and mRNA (messenger RNA; an intermediate molecule in protein production) must also be adhered to for the chosen host organism.

Also, different expression signals for the production of silk protein, and different vectors are required in different host organisms.

Naturally, optimal process conditions are designed to suit the host organism in question.

For example, the silk gene is likely to contain highly repetitive sequence information (a commonly occurring sequence in the protein, gly-ala-gly-ala-gly-ser, is paralleled by a repeating nucleotide sequence in the gene). Steps should be taken where possible to minimise the repetitiveness of the nucleotide sequence. This is because a repetitive nucleotide sequence can be subject to deletion or other rearrangement of genetic information in some hosts. This is of particular concern when growing large populations of organisms in a production process, since organisms with less extra genetic information (having suffered a deletion in the silk gene) can grow faster and overtake the population.

Recovery of the silk protein from the cells

The silk protein is recovered from the cells in which it is probably sorted in an insoluble form. The cells are broken up physically (eg mechanically) or chemically (eg enzymatically), and the silk protein is extracted and separated from the other cellular constituents. The silk protein can then be dissolved and spun to form fibre. If the silk protein is produced extra cellularly, it is purified from the fermentation medium.

## Claims

1. A method of producing components of silk, characterised by producing from a recombinant microorganism the protein fibroin for spinning to produce silk.

2. A method according to claim 1, further characterised by producing the protein sericin from a recombinant microorganism, for spinning together with fibroin.

3. A method according claim 1 or 2, characterised in that the microorganism(s) is(are) modified to produce modified protein(s), for producing silk having modified properties.

4. A method according to claim 1, 2 or 3, characterised in that wherein the silk protein comprises sets of the sequence $(Gly\text{-}Ala\text{-}Gly\text{-}Ala\text{-}Gly\text{-}Ser)_n$ forming crystalline regions.

5. A method according to claim 4, characterised in that the crystalline regions are interspersed with amorphous regions.

6. A method according to claim 4 or 5, characterised in that properties of the silk protein are varied by varying the length of the crystalline regions and/or varying the length of the amorphous regions.

7. A recombinant microorganism which has been genetically modified to be capable of producing fibroin and/or sericin.

8. A method of producing the components of silk such as fibroin and possibly also sericin by tissue culture techniques, for spinning to produce silk.

9. A method according to claim 8, characterised in that cells of Bombyx mori are grown in culture to produce the required silk components.

10. A method of growing cells of Bombyx mori in culture capable of producing fibroin, and possibly also sericin.

European Patent Office

# EUROPEAN SEARCH REPORT

Application number

EP 86 30 0434

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| A | CHEMICAL ABSTRACTS, vol. 88, no. 1, 2nd January 1978, page 320, abstract no. 3473j, Columbus, Ohio, US; J.F. MORROW et al.: "Studies on the silk fibroin gene", & MILES INT. SYMP. SER. 1977, 10(Recomb. Mol.: Impact Sci. Soc.), 409-17<br>--- | 1 | C 12 N 15/00<br>C 12 P 21/02<br>D 01 D 4/00 |
| A | CHEMICAL ABSTRACTS, vol. 88, no. 13, 27th March 1978, page 150, abstract no. 84885v, Columbus, Ohio, US; Y. OHSHIMA et al.: "Cloning of the silk fibroin gene and its flanking sequences", & PROC. NATL. ACAD. SCI. U.S.A. 1977, 74(12), 5363-7<br>--- | 1 | TECHNICAL FIELDS SEARCHED (Int. Cl.4) |
| A | CHEMICAL ABSTRACTS, vol. 88, no. 23, 5th June 1978, page 294, abstract no. 166571d, Columbus, Ohio, US; J.F. MORROW et al.: "Bacterial plasmids containing silk gene sequences", & MIAMI WINTER SYMP. 1977, 13(Mol. Cloning Recomb. DNA), 161-71<br>--- -/- | 1 | C 12 N |

The present search report has been drawn up for all claims

| Place of search | Date of completion of the search | Examiner |
| --- | --- | --- |
| THE HAGUE | 18-09-1986 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number

EP 86 30 0434

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | EXPERIENTIA, vol. 41, no. 9, September 1985, pages 1167-1171, Birkhäuser Verlag, Basel, CH; K. KIMURA et al.: "Molecular cloning of the fibroin light chain complementary DNA and its use in the study of the expression of the light chain gene in the posterior silk gland of Bombyx mori" * Whole document * | 1 | |
| | --- | | |
| A | NUCLEIC ACIDS RESEARCH, vol. 12, no. 3, 1984, pages 1543-1558, IRL Press Ltd., Oxford, GB; K. TOKUNAGA et al.: "In monkey COS cells only the TATA box and the cap site region are required for faithful and efficient initiation of the fibroin gene transcription" * Whole document * | 1 | TECHNICAL FIELDS SEARCHED (Int. Cl 4) |
| | --- | | |
| E | GB-A-2 162 190 (PA CONSULTING SERVICES) * Claims * | 1-10 | |
| | ----- | | |

The present search report has been drawn up for all claims

| Place of search | Date of completion of the search | Examiner |
|---|---|---|
| THE HAGUE | 18-09-1986 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82